# EUROPEAN PATENT APPLICATION

(11) **EP 4 166 112 A1**
(43) Date of publication of application: **19.04.2023**
(21) Application number: 21887891.6
(22) Date of filing: 10.11.2021
(51) Int. Cl.: A61F 2/28, A61F 2/30, A61L 27/50, A61L 27/14

(54) **SCAFFOLD FOR BONE REGENERATION AND CONSTRUCTION METHOD THEREFOR**

(30) Priority: 27.08.2021 KR 20210114218
(71) Applicant: Nanobiosystem Co., Ltd., Gwangju 61003 (KR)
(72) Inventor: SHIN, Kyoung Jin, Gwangju 61085 (KR); KIM, Sung Ho, Gwangju 61085 (KR)
(74) Representative: dompatent von Kreisler Selting Werner - Partnerschaft von Patent- und Rechtsanwälten mbB
(86) International application number: PCT/KR2021/016318
(87) International publication number: WO 2023/027247

(57) **Abstract**

The present invention provides a scaffold for bone regeneration and a method of manufacturing the same. The scaffold for tissue regeneration of the present invention has a radial pattern including grooves and ridges on one surface thereof, thereby providing an environment suitable for cell attachment and migration, as well as bone differentiation. Therefore, the scaffold may be effectively used as a material for tissue regeneration.

## Description

### [Technical Field]

The present invention relates to a scaffold for bone regeneration and a method of manufacturing the same.

### [Background Art]

Tissue regeneration abilities of a human body are very complex, and self-regeneration abilities are very limited because of a subtle tissue structure thereof. The human body has the possibility of regeneration at the time of tissue damage due to various cells including stem cells and constitutive environments, but for reasons such as an accident, disease, aging, and the like, a case of exceeding the limits of regenerative function may occur. Due to an increase of various diseases and accidents together with growth of aging populations in recent years, a necessity for regeneration of organ tissues is increasing rapidly as the organ tissues of a living body are being pushed beyond the limits of the bodies regeneration abilities.

In particular, bone tissue is an important tissue to maintain a skeleton of the human body, and various materials and types of bone graft materials for bone tissue replacement and regeneration are being researched and developed for bone tissue regeneration. The bone graft material may be classified into a bone forming material, a bone conductive material, and a bone growth inducing material depending on the bone healing mechanism. In bone grafting, methods such as autologous transplantation, allogenic transplantation, heterogenic transplantation, etc. are mainly used depending on the graft material used for bone transplantation or implantation.

Among them, the autologous transplantation is a method capable of minimizing an immune response, and has an advantage of enabling the bone tissue to be stably regenerated by minimizing the immune response when transplanting autologous bone to a bone damaged site. However, the autologous transplantation causes inconveniences such as a secondary bone loss in other sites and a long recovery period due to the collection of the autogenous bone, and has a disadvantage that an available amount is very limited. To compensate for these problems, there is a heterogenic transplantation using another person's bone, but unlike the autologous bone, it causes many immune responses and has a disadvantage of being very expensive. Accordingly, a lot of research on bone tissue engineering to manufacture and transplant synthetic bone intended to be used in many people while minimizing the immune response is being proceeded.

Tissue engineering is a field of regenerative medicine ranging from cells to artificial organs, and is recognized as an important technology in life sciences and medical fields in the future based on studies of biological and engineering technologies from biomaterials to inorganic materials, which may help the restoration of tissues or organs. Various methods are being studied in order to understand the correlation between the structure and function of the living body, and furthermore, achieve purposes of restoring, maintaining and improving the functions of the human body by making substitutes for living bodies and then implanting the same into the body. One of key technologies in tissue engineering is to create a support or scaffold which plays a role of supporting so that cells can grow well. Unlike a two-dimensional membrane or capsule, the scaffold has a three-dimensional shape, and refers to a space in which all cells in the body having a three-dimensional structure may be attached thereto, and then differentiated and proliferated.

The scaffold plays a very important role in bio-tissue engineering. In detail, the scaffold plays an important role in the growth of cells seeded in a porous structure and cells migrating from the surrounding tissues. Most cells in the human body are adherent cells that grow with being attached, and if there is no place to attach, the cells may not grow and die. Therefore, the scaffold should provide an environment suitable for attachment, differentiation, growth, and migration of cells.

Accordingly, the present inventors have confirmed that a scaffold having radial topographic features promotes cell behavior and bone regeneration, and therefore, the present invention has been completed on the basis of the above confirmation.

### [Summary of Invention]

### [Problems to be solved by Invention]

It is an object of the present invention to provide a scaffold for bone regeneration.

Another object of the present invention is to provide a method of manufacturing a scaffold for bone regeneration.

### [Means for Solving Problems]

A scaffold for tissue regeneration including: a radial pattern which includes a plurality of grooves and ridges on one surface thereof.

The scaffold for tissue regeneration according to the above 1, wherein the radial pattern is not formed in a central region of the scaffold.

The scaffold for tissue regeneration according to the above 1, wherein the radial pattern has a width of 0.1 um to 100 um.

The scaffold for tissue regeneration according to the above 1, wherein a space between the radial patterns is 0.1 um to 100 um.

The scaffold for tissue regeneration according to the above 1, wherein the scaffold has a circular shape.

The scaffold for tissue regeneration according to the above 1, the scaffold includes: a substrate; and a pattern layer having the pattern on the substrate.

The scaffold for tissue regeneration according to the above 1, wherein the pattern layer includes at least one material selected from the group consisting of a polycaprolactone (PCL) resin, a polyurethane acrylate (PUA) resin, a polyvinyl alcohol resin, a polyethylene resin, a polypropylene resin, a polyethylene glycol resin, a poly(L-lactide-co-glycolide) resin, a polylactic acid (PLA) resin, a polyglycolic acid (PGA) resin, chitosan, gelatin, collagen, and a combination thereof.

A method of manufacturing the scaffold for tissue regeneration according to the above 1, the method including: coating a glass with a polymer at a predetermined thickness; pressing the coated polymer with a mold on which a radial pattern is formed to form a pattern; and separating the polymer from the glass after the pattern is formed.

The method according to the above 8, wherein the coating is spin coating in which the polymer is placed on the glass, and then the polymer is adhered to the glass by rotating the glass.

The method according to the above 8, wherein the step of forming the pattern includes: heating the coated polymer; and pressing the heated polymer with the mold having a radial pattern formed thereon, followed by cooling the same.

The method according to the above 8, further including: heating the coated polymer before the step of forming the pattern; pressing the heated polymer with a flat mold to flatten a surface thereof; and cooling the flattened polymer.

The method according to the above 10 or 11, wherein the cooling is performed by spraying air to the glass.

The method according to the above 8, wherein the separating step is performed by immersing the polymer coated glass in ethanol.

The method according to the above 8, further including drying the separated polymer.

### [Advantageous effects]

According to the present invention which relates to a scaffold for tissue regeneration and a method of manufacturing the same, the scaffold for tissue regeneration of the present invention has a radial pattern including grooves and ridges on one surface thereof, thereby providing an environment suitable for cell attachment and migration, as well as bone differentiation. Therefore, the scaffold may be effectively used as a material for tissue regeneration.

### [Brief Description of Drawings]

FIG. 1 is a schematic view illustrating a manufacturing process of polycaprolactone (PCL) scaffold having a radial pattern.
FIG. 2 is FE-SEM images illustrating surfaces of three types (planar, aligned, and radial) of PCL scaffolds.
FIGS. 3 to 5 are views illustrating analysis equipment and results of mechanical properties of the three types (planar, aligned, and radial) of PCL scaffolds.
FIG. 6 is a schematic view illustrating the effect of radial topography on cell morphology, proliferation and differentiation.
FIG. 7 is immunofluorescence images of osteoblast-like cells cultured in the three types (planar, aligned, and radial) of PCL scaffolds.
FIG. 8 is graphs illustrating results of quantitative analysis of cell bodies of cells grown in the three types (planar, aligned, and radial) of PCL scaffolds.
FIG. 9 illustrates results of quantitative analysis of cell adhesion and proliferation in the three types (planar, aligned, and radial) of PCL scaffolds.
FIG. 10 graphs illustrating results of analysis of bone differentiation in the three types (planar, aligned, and radial) of PCL scaffolds.
FIG. 11 is a schematic view illustrating regenerative effects of radial topography in a bone defect site.
FIGS. 12 to 14 are views for comparing tissue regeneration abilities of the three types (planar, aligned, and radial) of PCL scaffolds with commercially available products in a calvarium defect site in mice.
FIGS. 15 to 20 are views illustrating results of confirming cell migration and growth factor secretion in the radial PCL scaffolds.

### [Mode for Carrying out Invention]

Hereinafter, the present invention will be described in detail.

The present invention relates to a scaffold for bone regeneration having a radial pattern which includes a plurality of grooves and ridges on one surface thereof.

The scaffold of the present invention may include grooves and ridges, thereby having a structure similar to the topographical characteristics of an extracellular matrix (ECM) of bone tissue.

The number of grooves and ridges is not particularly limited, and may be appropriately selected depending on types of tissues to be regenerated and the number or density of cultured cells.

The scaffold of present invention is provided with a radial pattern. Specifically, a plurality of grooves and ridges are arranged in a radial pattern, such that cells may be aligned/oriented in a central direction, and thereby regulating cell migration and cell elongation, as well as regulating cell organization and promoting tissue regeneration.

Shapes of the pattern are not particularly limited so long as they allow cells to be arranged in a certain direction, and may be, for example, shapes whose longitudinal sections are each independently triangular, square, pentagonal, circular, or elliptical, but preferably, the shapes thereof are a square in terms of a more regular cell arrangement.

The pattern may be a nano pattern or a micro pattern.

The width of the pattern may be appropriately selected so as to have a width similar to the arrangement of the extracellular matrix (ECM) of the tissue to be regenerated. For example, the micro pattern may have a width of 0.1 µm to 100 pm, 0.5 µm to 80 pm, or 1 µm to 50 um. More specifically, the width of the ridge may be constant or may be gradually increased from a central portion toward an edge. When the width of the ridge is increased toward the edge, for example, the central portion may have a width of 0.1 um to 5 um, and the edge may have a width of 1 um to 10 µm, but it is not limited thereto. In addition, the width of the groove may be gradually increased from the central portion toward the edge. For example, the central portion may have a width of 0.1 um to 10 um, 0.5 um to 8 um, or 1 um to 6 um, and the edge may have a width of 20 um to 100 um, 30 um to 80 um, or 40 um to 50 um, but it is not limited thereto. For example, the nano pattern may have a width of 100 nm to 900 nm, 300 nm to 800 nm, or 400 nm to 700 nm. More specifically, the width of the ridge may be constant or may be gradually increased from the central portion toward the edge. When the width of the ridge is increased toward the edge, for example, the central portion may have a width of 100 nm to 300 nm, and the edge may have a width of 200 nm to 400 nm, but it is not limited thereto. In addition, the width of the groove may be gradually increased from the central portion toward the edge. For example, the central portion may have a width of 100 nm to 300 nm, 150 nm to 250 nm, or 180 nm to 230 nm, and the edge may have a width of 600 nm to 900 nm, 650 nm to 850 nm, or 700 nm to 800 nm, but it is not limited thereto.

In addition, a space between the patterns may be appropriately selected so to have a structure similar to the arrangement of the extracellular matrix of the bone tissue to be regenerated. For example, the adjacent micro patterns may have a space of 0.1 um to 100 um, 0.5 um to 80 um, or 1 um to 50 um. More specifically, the space between the patterns may be gradually increased from the central portion toward the edge. For example, the adjacent central portions may have a space of 0.1 um to 10 um, 0.5 um to 8 um, or 1 um to 6 um, and the adjacent edges may have a space of 20 um to 100 um, 30 um to 80 um, or 40 um to 50 um, but it is not limited thereto. For example, the adjacent nano patterns may have a space of 100 nm to 900 nm, 300 nm to 800 nm, or 400 nm to 700 nm. More specifically, the space between the patterns may be gradually increased from the central portion toward the edge. For example, the adjacent central portions may have a space of 100 nm to 300 nm, 150 nm to 250 nm, or 180 nm to 230 nm, and the adjacent edges may have a space of 600 nm to 900 nm, 650 nm to 850 nm, or 700 nm to 800 nm, but it is not limited thereto.

The scaffold of the present invention may variously control topographical density by variously adjusting the widths and space of the patterns. Accordingly, the scaffold has a density similar to the arrangement of the extracellular matrix of the tissue to be regenerated, such that the cells may be arranged in accordance with the target tissue, and cell characteristics such as a cell migration velocity, cell elongation, stress fiber distribution, cytoskeleton, size of focal adhesion (FA), length of fibronectin fiber bundle or wound dressing rate may be controlled.

In the scaffold of the present invention, the radial pattern is not formed on a central region thereof. Since the pattern is formed at a location spaced apart from a center of the scaffold at a predetermined interval, the central region having no radial pattern formed thereon provides a central space where the cells migrated in the central direction of the scaffold can be gathered, such that tissue regeneration may be promoted.

The interval of the pattern spaced apart from the center of the scaffold may be, for example, 10 um to 60 um, 15 um to 50 um, or 20 um to 40 um, but it is not limited thereto.

The scaffold of the present invention may have a circular shape. When the scaffold has the circular shape, the pattern is constantly arranged, such that the cells may be more uniformly aligned than the case of having other shapes (e.g., rectangular or oval). Accordingly, in the bone tissue to be regenerated, cells may uniformly grow and proliferate without being biased to a specific region, such that tissue regeneration can be effectively achieved.

The scaffold of the present invention may include a pattern layer having the radial pattern. For example, the scaffold of the present invention may include a substrate and a pattern layer having the pattern on the substrate.

Any material known in the art, such as glass and polymer, may be used for the substrate without limitation thereof.

The pattern layer is located on the substrate.

The pattern layer may include at least one material selected from the group consisting of, for example, a polycaprolactone (PCL) resin, a polyurethane acrylate (PUA) resin, a polyvinyl alcohol resin, a polyethylene resin, a polypropylene resin, a polyethylene glycol resin, a poly(L-lactide-co-glycolide) resin, a polylactic acid (PLA) resin, a polyglycolic acid (PGA) resin, chitosan, gelatin, collagen, and a combination thereof. Preferably, in terms of biocompatibility, the polycaprolactone (PCL) resin, the polyurethane acrylate (PUA) resin, and the poly(L-lactide-co-glycolide) resin are used, and more preferably, in terms of tissue regeneration, the polycaprolactone (PCL) resin is used, but it is not limited thereto.

For example, the pattern layer may be formed in such a manner that: ridges are formed on the substrate at a predetermined interval, and then valleys are formed between the ridges; valleys are formed on the substrate at a predetermined interval, and then ridges are formed between the valleys; or a pattern layer material is applied to the substrate, and then the substrate is pressed with a mold on which a pattern is formed, but it is not limited thereto.

If necessary, an adhesive layer may be further included between the substrate and the pattern layer.

A material for the adhesive layer is not particularly limited so long as it can adhere the substrate and the pattern layer, and for example, a combination of phosphoric acid acrylate and propylene glycol monomethyl ether acetate may be used.

The present invention provides a method of manufacturing the scaffold for bone regeneration.

The method of the present invention includes coating a glass as a substrate with a polymer at a predetermined thickness.

The coating may be performed using a known means by a method under conditions known in the art. For example, the coating may be spin coating in which the polymer is placed on the glass, and then the polymer is adhered to the glass by rotating the glass, but it is not limited thereto.

The method of the present invention includes pressing the coated polymer with a mold on which a radial pattern is formed to form a pattern.

The step of forming the pattern may be performed, for example, by capillary force lithography (CFL).

Capillary force lithography is a method of forming a pattern by pulling up a polymer into an empty space of a mold using a natural force called capillary action, and may form a pattern in fine and precise manner.

As the mold, for example, a polyurethane acrylate (PUA) mold or a polydimethylsiloxane (PDMS) mold may be used. The PUA (Young's modulus: 100-400 MPa) mold and the PDMS (Young's modulus: 2 MPa) mold have different Young's modulus, such that it is possible to select the mold depending on the material from which the pattern is to be manufactured using capillary force lithography. When forming a pattern using the polyurethane acrylate (PUA) resin, the polycaprolactone (PCL) resin, or the poly(L-lactide-co-glycolide) resin, more delicate patterns (patterns having narrower space therebetween) may be prepared by using a PUA mold. Therefore, the present invention may use the PUA mold.

The step of forming the pattern may include heating the coated polymer; and pressing the heated polymer with the mold having a radial pattern formed thereon, followed by cooling the same.

The method of the present invention may further include: heating the coated polymer before the step of forming the pattern; pressing the heated polymer with a flat mold to flatten a surface thereof; and cooling the flattened polymer.

The heating temperature is not particularly limited, and may be appropriately adjusted depending on the melting temperature of the polymer.

The cooling may be performed using a known means by a method under conditions known in the art. For example, the cooling may be performed by spraying air to the glass, but it is not limited thereto.

The method of the present invention includes separating the polymer from the glass after the pattern is formed.

The separating step may be performed using a known means by a method under conditions known in the art. For example, the separating may be performed by immersing the polymer coated glass in ethanol, but it is not limited thereto.

If necessary, the method may further include drying the separated polymer.

The drying may be performed in a conventional dryer such as a tunnel-type dryer, a box-type dryer, a spray dryer, and the like, for example.

Hereinafter, preferred embodiments will be described to more concretely understand the present invention.

### 1. Materials and methods

### (1) Design and fabrication of the radially patterned PCLbased scaffold

Radially patterned PCL scaffolds imprinted directly onto silicon wafers are not easily peeled off due to the surface charge of silicon wafers. Therefore, we used a polydimethylsiloxane(PDMS) mold. First, the PDMS prepolymer Sylgard 184 Silicon Elastomer (Dow Corning) was mixed with 10% curing agent, poured into a petridish to a sufficient thickness, and baked at 70 °C for at least 2 h to ensure curing without any residue. The polymer was then peeled off from the petri dish and oxidized for 5 min and 15 min at a wavelength 100 nm using an UV treatment system. To ensure uniform oxidation, the distance of the polymer from the UV lamp was constant at 5 cm. The UV/ozone dose was 15 mW/cm2(measured at a distance of 10mm at wavelengths of 185 nm and 254 nm). Thereafter, 100 mL of 10% (w/v) adhesion promoter 3-(trimethoxysilyl) propyl methacrylate (TMSPMA) was drop-dispensed onto the UV/ozone-treated PDMS sheet. To replicate radial structures (5 µm wide and deep) on PDMS, a polyurethane acrylate (PUA) precursor (Changsung Sheet, Korea) was drop-dispensed and UV-cured for use as a selfreplicating mold. Finally, additional UV-curing was performed for more than 10 h to remove any non-cured acrylate groups. The UV assisted PUA precursor solution was dropped onto the silicon master mold, which was then covered with 100-µm thick poly (ethyleneterephthalate) film (SKC, Korea). For curing, the master mold covered with the PUA precursor was exposed to UV light (λ = 310-400 nm, 40 W) for 30 s.

PCL beads (Mw: 80,000; Sigma Aldrich, USA) were dissolved in dichloromethane (18% wt.) by mixing for 1d using a magnetic stirrer. To fabricate the PCL scaffold, PCL solution was poured onto a clean square piece of glass (18mm× 18 mm) and spin-coated at 3500 rpm for 240 s. The glass coated with PCL was then pressed at ~70 kPa with flat PDMS fabricated on a hot plate at 80 °C. Following the imprinting process, the fabricated flat PCL scaffolds were cooled to room temperature and the PDMS mold peeled from the PCL scaffolds. Using the same process, the aligned and radial PCL scaffolds were pressed at ~ 70 kPa with aligned and radial patterned PDMS, respectively, that were fabricated on a hot plate at 70 °C. Likewise, the aligned and radial patterned scaffolds were cooled to room temperature and the PDMS mold peeled from the aligned and radial PCL scaffolds.

### (2) Scaffold characteristics and properties analysis

Field emission scanning electron microscopy (FE-SEM) images of the surfaces of various patterned scaffolds fabricated in the study were captured using a JSM7500F FE-SEM microscope (Oxford, UK) at an acceleration voltage of 15.0 kV and average working distance of 8.8 mm. The samples were coated with platinum prior to morphological observation. Chemical characteristics of the flat, aligned, and radial scaffolds were analyzed to confirm their chemical variations. The chemical bond structures were examined by Fourier-transform infrared spectroscopy (FT-IR) using a FT-IR spectrometer(Spectrum 400, USA).

The mechanical strain and stress tests of the various patterned PCL scaffolds were performed using MCT-1150 tensile testers (A&D Company, Japan) at a test speed of 100 mm/min. The tests included the analysis of 10 specimens per sample with the same interval set. Normal and shear adhesion forces of various patterned PCL scaffolds were evaluated using porcine rind and measured using an MCT-1150 instrument at a test speed of 50 mm/min. Prior to the adhesion test, a fresh porcine rind was rinsed with deionized water, the various patterned PCL scaffolds were attached to its surface, and rind/scaffolds measured under a preload of ~ 0.5/cm2. The pulling weight was gradually increased until the adhesion force felled off.

### (3) Immunofluorescence

Adherent cells on various patterned PCL scaffolds were fixed with a 4% paraformaldehyde solution (Biosesang, Korea) for 15 min and permeabilized with 0.2% Triton X-100 (Biosesang). The fixed cells were then blocked with 3% normal goat serum (NGS; Abeam, Cambridge, MA, USA) in phosphate-buffered saline (PBS; Biosesang). After being washed with PBS, the scaffolds were stained with tetramethylrhodamine (TRITC)-conjugated phalloidin (Millipore) for 1 h and then with 4, 6-diamidino-2-phenylindole (DAPI; Millipore) for 3 min. Images of the stained cells were obtained using a fluorescence microscope. For quantitative analysis of the morphology of the MG-63 cells on the substrates, images were obtained using a fluorescence microscope and analyzed using Image J software.

### (4) Cell attachment and proliferation analysis

We quantified the cell attachment using the WST-1 assay, which is the same method as previous studies. Specifically, Osteoblast-like MG-63 cells (1 × 10⁴ cells/samples) were seeded onto the scaffold samples and cultured in Dulbecco's Modified Eagle Medium (DMEM) containing 10% fetal bovine serum (FBS) and 1% antibiotics (Cellgro, USA) for 6 h, 3 d and 5 d at 37 °C in a humidified atmosphere containing 5% CO2. Quantitative analysis of the proliferation of cells growing on the samples was performed by WST-1 assay using a Premix WST-1 Cell Proliferation Assay System (Takara Bio Inc., Kusatsu, Japan). To confirm cell attachment prior to performing the cell proliferation assays, the scaffolds were washed with PBS to remove any non-adherent cells.

### (5) Osteogenic mineralization analysis

Osteoblast-like MG-63 cells (4 × 10⁴ cells/sample) were cultured for 7 d or 14 d on scaffold samples in osteogenic differentiation medium (100 nM dexamethasone, 50 µm ascorbic acid, and 10mm glycerol 2-phosphate in DMEM). After 7 d, the cells were washed with PBS and fixed in 4% paraformaldehyde for 15 min. The fixed cells were stained with Alkaline Phosphatase Blue Membrane Solution (Sigma Aldrich, USA) to confirm early osteogenic differentiation of the osteoblasts on the scaffold surfaces. The stained cells were then de-stained using a SensoLyte pNPP Alkaline Phosphatase Assay Kit (Ana-Spec, Inc., USA) and the extracted stain then measured using a microplate reader at 405 nm to quantify the osteogenic differentiation of the osteoblasts. In addition, MG-63 cells were stained with Alizarin Red S (Sigma Aldrich, USA) after 14 d of culturing to confirm later osteogenic differentiation according to the degree of mineralization of the MG-63 cells on sample surfaces. To quantify the osteogenic differentiation of the MG-63 cells, the Alizarin Red S stained cells were then destained with cetylpyridinium chloride (Sigma Aldrich) and the extracted stains quantitated using an iMarkTM Microplate Absorbance Reader (Bio-Rad, Hercules, CA, USA) at 595 nm.

### (6) In vivo animal study

The animal study was approved by the Ethics Committee of Chonnam National University. Six-week-old male C57Bl/6 N mice were assigned to six different groups (n = 5/group): Defect (control), flat, aligned, radial, and collagen-based products (Geistlich Bio-Gide; Geistlich Pharma, Switzerland). The mice were fully anesthetized with an intraperitoneal injection of Zoletil (0.006 cc/10g) and Rumpun (0.004 cc/10 g). The heads of the mice were then shaved and disinfected. The bones were exposed by incising the skin approximately 3.0 cm above the calvaria bone. Bone defects (diameter: 5 mm) were generated on the one side of the exposed calvaria bone using an electric drill. Prepared scaffolds (Diameter: 5 mm) were placed on the calvarial bone defect. After suturing the skin, the ambient temperature was raised and the mice were woken from the anesthesia. The mice were euthanized 4 wk. or 6 wk. after surgery and tissues obtained, including the calvaria bone and defect region. Calvaria bone microcomputed tomography (µ-CT) was performed using a Skyscan 1172 Micro-CT (Skyscan, Konitch, Belgium) at a resolution of 11.38 pixels and exposure time of 316 ms, with an energy source of 80 kV and current of 124 µA. An average of 488 slices of calvaria bone were scanned. The µ-CT images were analyzed using MIMICS 14.0 3D imaging software (Materialise's Interactive Medical Image Control System, Leuven, Belgium). The calvaria bone specimens were fixed in 10% formalin and decalcified in 0.5M ethylenediaminetetraacetic acid (pH 7.4) at room temperature for 7 d. After the specimens were embedded in paraffin, they were cut into 5-mm thick sections and then stained with hematoxylin and eosin (H&E). Images were obtained using Aperio Images-Scope software (Leica, CA, USA).

### (7) Migration assay

A thin PDMS slab was used to provide a cell-free area for investigating the migration and wound-healing effects of osteoblasts on the samples. Specifically, a 5mm thick PDMS sheet was cut into 5mm round slabs using a steel punch. The PDMS slabs were placed onto the scaffold surfaces. MG-63 cells (1 × 10⁵cells/samples) were cultured on the woundgenerated samples. The PDMS slabs were then manually removed using sharp tweezers and the cells of the wound were evaluated by immunofluorescence 24 h post-culturing using a fluorescence microscope (Zeiss, Germany). The cellcovered area and cell migration rate in the wound were evaluated using Image J.

### (8) Western blotting

For western blot analysis, RIPA Cell Lysis Buffer (Biosesang) containing Xpert Proteinase Inhibitor Cocktail (GenDEPOT, Houston, TX, USA) was used to lyse cells. Protein from the lysed cells was incubated on ice for 30 min and then centrifuged at 13,000 rpm for 30 min at 4 °C. Protein concentrations were quantitated using a DC Protein Assay Kit (Bio-Rad, Hercules, CA, USA) and the Lowry assay. Equal amounts of proteins were loaded onto sodium dodecyl sulfate polyacrylamide gel electro-phoresis gels, the proteins were resolved by electrophoresis, and then transferred onto polyvinylidene difluoride membranes (IPVH00010; Millipore) using an electro-blotting apparatus at a constant voltage of 30 V for 1 h. The membranes were blocked for 1 h with 5% skim milk in Tris-buffered saline containing Tween 20 (TBST). Subsequently, the membrane was incubated with the following primary antibodies overnight at 4 °C: extracellular signal-related kinase (ERK, 1:1000; Santa Cruz Biotechnology, CA, USA), focal adhesion kinase (FAK, 1:1000; Cell Signaling Technology, Beverly, MA, USA), and glyceraldehyde 3-phosphate dehydrogenase (GAPDH, 1: 5000; Abcam). Goat anti-mouse IgG (H + L)-HRP and goat anti-rabbit IgG (H + L)-HRP (GenDEPOT, Houston, TX, USA) were used as secondary antibodies at a dilution of 1:10,000 to probe the membrane for 1 h at room temperature. The membranes were then washed three times for 10 min each in TBST. Bands were detected using West Pico PLUS Chemiluminescent Substrate (Thermo Scientific, Waltham, MA, USA) .

### (9) Growth factors assay

To ascertain whether the MG-63 cells could secrete growth factors and cytokines on the radially surface, the cells (3 × 10⁴ cells/samples) were seeded on the substrates and cultured for 5 days in the proliferation medium. The cells were then cultured for 3 days in the osteogenic differentiation medium. Finally, the cells were again cultured for 1 day in DMEM. using the RayBio GSeries Human Growth Factor Array 1 Kit (RayBiotech, Norcross, GA) according to the manufacturer's protocol. The assay kit was first treated with blocking buffer for 30 min. Then the culture medium containing the secreted proteins was incubated with the kit for 2 h at room temperature. After washing with wash buffer, a biotinylated antibody cocktail was added for 2 h, following which the kits were washed several times. Cy3 equivalent dye-conjugated streptavidin was then incubated with the kit for 1 h, following which washing was carried out several times. Fluorescence images of the reacted kits were obtained using the GenePix 4000A (Molecular Devices).

### (10) Statistical analysis

All quantitative data are presented as the mean ± standard deviation. Unpaired Student t-tests were used for statistically analyzing the results of cell adhesion, viability, and differentiation experiments. To compare three or more conditions, one-way ANOVA was performed. P-values less than 0.05 were considered significant. Micro-CT scans were subjected to the Kruskal-Wallis test for statistical analyses using the SPSS software.

### 2. Results

### (1) Design and characteristics of the PCL flat, aligned and radial patterned scaffolds

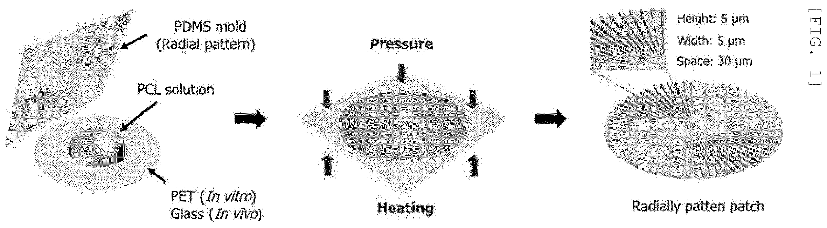

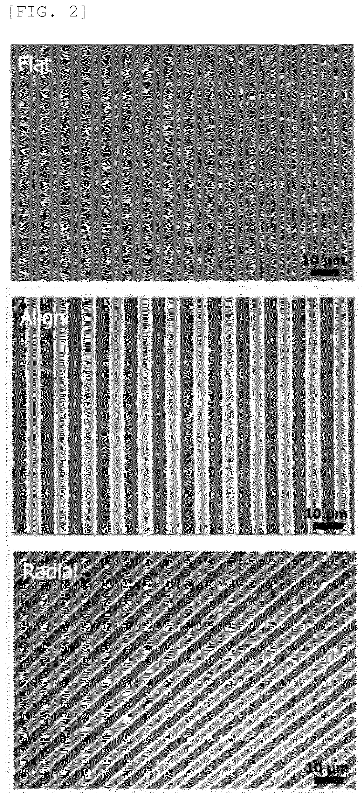

A schematic of the fabrication process of the radial patterned PCL scaffolds using lithography technology used in this study is shown in FIG. 1. The FE-SEM images of the surface morphology of the three different types of scaffolds (flat, aligned, and radial) revealed different topographical orientations. The align and radial patterned scaffolds show the aligned topography along the pattern direction compared to flat patterned scaffolds. As shown in the FIG 2, the diameter of the pattern and the spacing between patterns are equal to 5 µm in align and radial pattern; however, the direction of the two patterns is different. Specifically, the radial scaffold showed a align pattern direction toward the center. To confirm the maintenance of the chemical properties of the three types of scaffolds, functional groups of the scaffolds were evaluated by FT-IR spectroscopy (Supporting Fig. 1). The characteristic absorption bands related to PCL (CH2 asymmetric stretching at 2944 cm-1, symmetric stretching at 2866 cm-1, C=O stretching vibration of carbonyl groups at 1721 cm-1, and deformation of C-O at 1161 cm-1) were detected for all scaffolds.

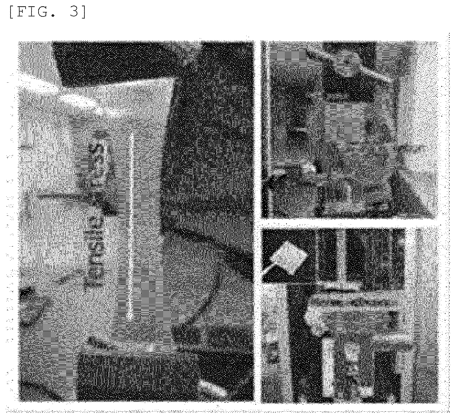

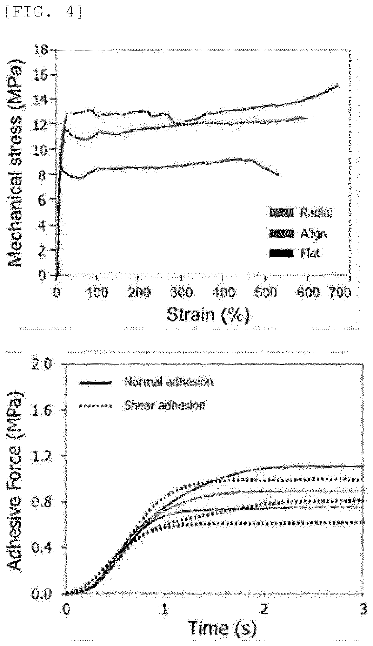

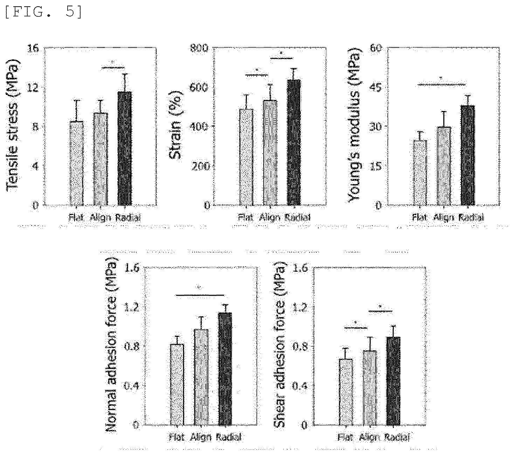

To confirm the mechanical properties of the three types of fabricated scaffolds, we investigated the effect of different topographies on the mechanical properties of the scaffolds by calculating the strain, stress, and Young's modulus from stress-strain curves (FIGs. 3 and 4). The tensile strength and strain of the radial patterned scaffolds (~ 11.6MPa and 630%, respectively) were higher than those of the aligned patterned scaffolds (~ 9.8 MPa and 560%, respectively) and flat patterned scaffolds (~ 8.1MPa and 490%, respectively). Moreover, the Young's modulus value for the flat and aligned patterned scaffolds was 23.2 and 31.2MPa, respectively, whereas that for the radial patterned scaffolds was 38.4MPa (FIG. 5).

To analyze the effects of different topographies on the tissue-bonding performance of the scaffolds, we compared the normal and shear adhesive forces among the three scaffold types (FIG. 3). As shown in FIGs 4 and 5, the radial patterned scaffolds exhibited higher normal adhesive force (114 kPa) than that of the aligned patterned scaffolds (92 kPa) and the flat patterned scaffolds (81 kPa). Similarly, the radial patterned scaffolds displayed higher shear (85 kPa) and adhesive forces compared to those of the aligned patterned scaffolds (66 kPa) and flat patterned scaffolds (59 kPa). On the basis of these results, we demonstrated the mechanical properties of radially patterned PCL scaffolds are improved than those of previously reported patch-type non-patterned and fibrous PCL-based scaffolds.

### (2) In vitro analysis of cell behavior on flat, aligned, and radial patterned PCL scaffolds

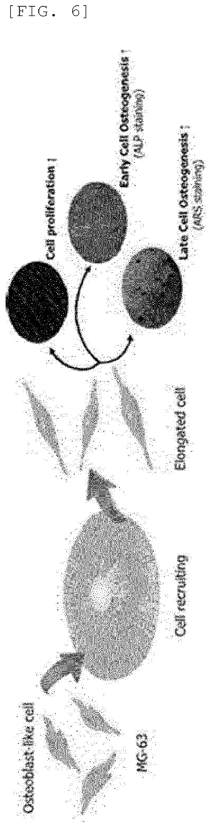

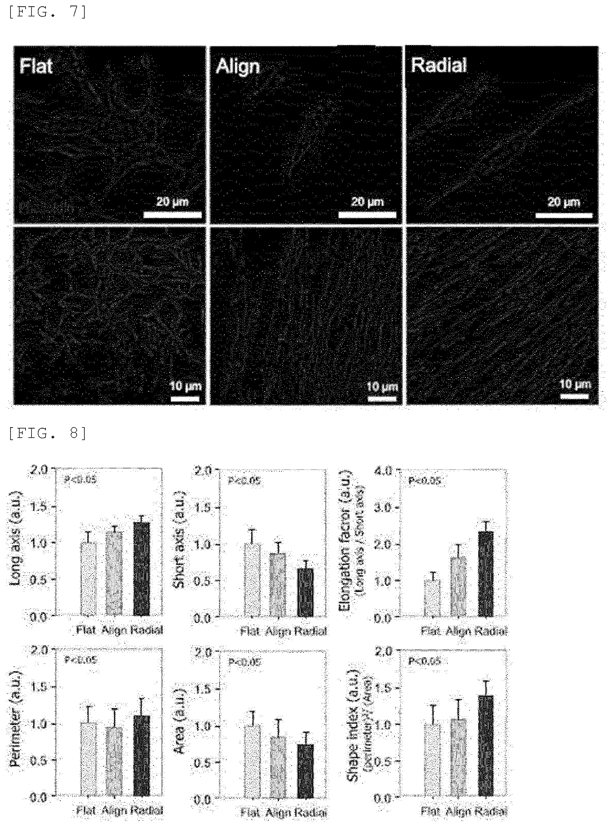

It is well known that topographical structure of the scaffolds affects cellular morphology and function. Immunofluorescence was used to investigate the effects of the different topographical cues on shape and orientation at a single-cell level. As shown in FIG. 6, the radial topographical cue greatly influenced cell polarity, as indicated by the aligned cytoskeletal structure of cells. Cells on grown on structures with radial topography were more elongated compared to those grown on structures with aligned and flat topographies (FIG. 6). The cells grown on structures with flat topography (un-patterned surface) showed relatively spherical shapes compared to those grown on structures with aligned and radial topographies (FIG. 7). Cell elongation factors (CEFs), defined as (major axis)/(minor axis), were calculated to quantify the observed elongation of cells. Surprisingly, the CEF of cells grown on radial topography was approximately twice greater than that of cells grown on flat topography (FIG. 8). Meanwhile, the area of cell body spread decreased on the radial topography (FIG. 8). Here, we introduced the cell shape index (CSI), which was calculated as follows: CSI=(cell perimeter)²/(cell area). With the increasing trend of CEF, higher CSI values observed on the radial topography, indicating the cell body may be closely interconnected to each other in order to regulate their functions.

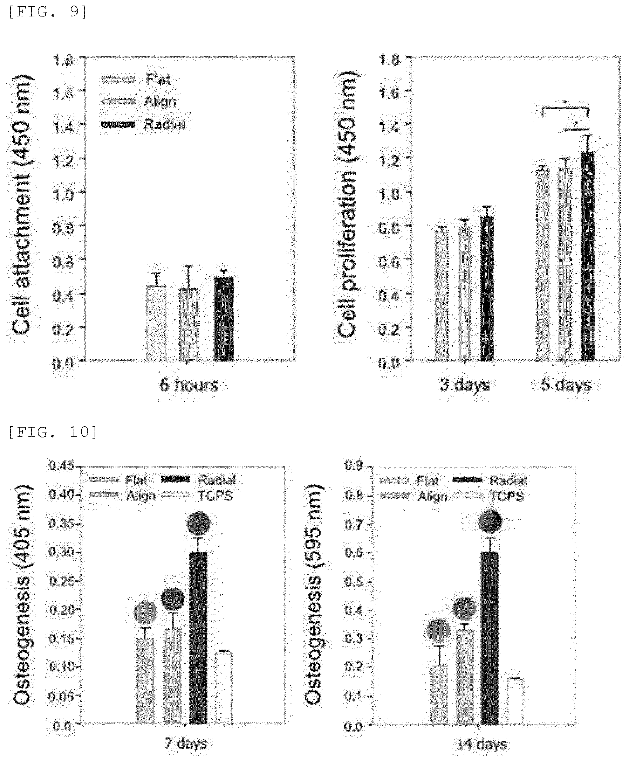

To investigate whether different topographies influenced cell proliferation and cell attachment, we cultured MG-63 cells on the three types of scaffolds for 6h (cell attachment assay), 3 d (cell proliferation assay), and 5 d (cell proliferation assay) (FIG. 9). After 6h of culture, unattached cells were removed by washing with PBS and adhered cells on the scaffolds were quantified by WST-1 assays. We determined MG-63 cells were well attached on all scaffolds, irrespective of topographic properties. However, cells grown on the radial patterned scaffolds showed higher attachment than those of the aligned patterned scaffolds and flat patterned scaffolds. After 3 d and 5 d of culture, cell proliferation was greater on the radial patterned scaffolds compared with those of the other two scaffolds (FIG. 9).

Phosphatase deposition of osteoblast-like cells on the three scaffold types was also examined by culturing MG-63 cells on the scaffolds in osteogenic induction medium for 7 d. Alkaline phosphatase staining revealed higher osteogenic levels on the radial patterned scaffolds compared to that on the aligned patterned scaffolds, the flat patterned scaffolds, and the tissue culture polystyrene substrate (TCPS). Osteogenic mineralization of MG-63 cells on the three types of scaffolds was also examined by culturing the cells on the scaffolds in osteogenic induction medium for 14 d. Alizarin Red S staining (FIG. 10) revealed higher calcium expression levels on the radial patterned scaffolds compared to that on the aligned patterned scaffolds, the flat patterned scaffolds, and the TCPS. The alkaline phosphatase staining (early osteogenic differentiation) and Alizarin Red S staining (late osteogenic differentiation) demonstrated improved bone differentiation in the radial patterned scaffolds compared to that observed in the other two scaffolds.

### (3) Bone tissue regeneration in vivo

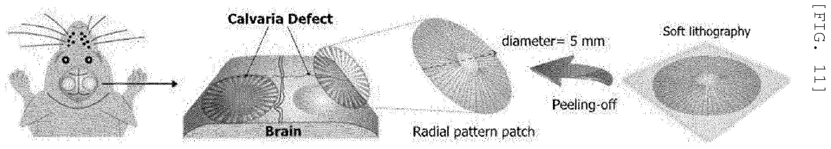

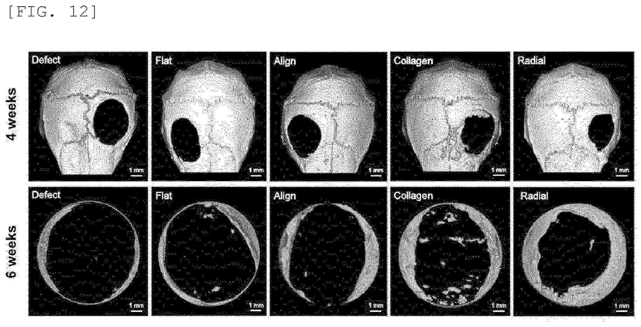

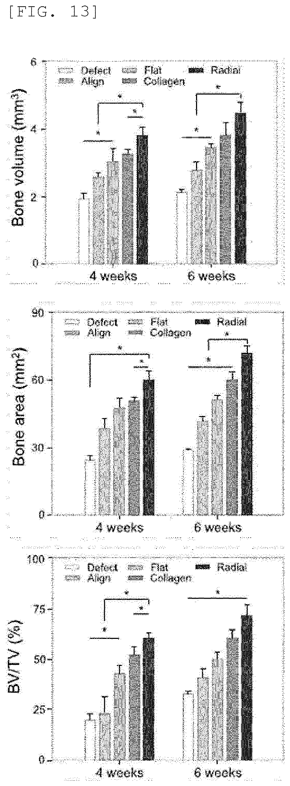

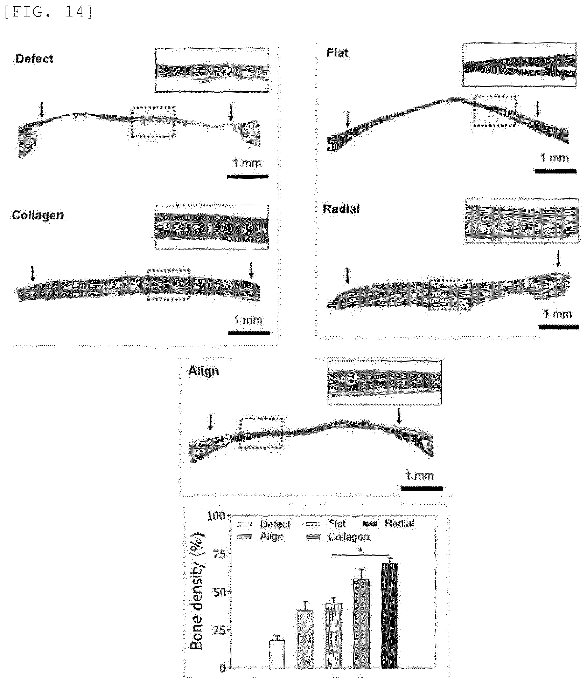

All mice used in the in vivo studies survived till the euthanization date and no adverse reactions were observed. The three scaffold types (5 mm diameter) and collagen-based commercial products were placed on the surgically induced bone defect. No infection or inflammatory reaction was observed in any of the mice at any point during the postoperative period. The in vivo bone regeneration results confirmed the effects of radial topography (FIG. 11). The scaffolds persisted for 6 wk. without deformation. Quantitative assessment of the effects of radial topography on bone formation was performed on new bone defects in vivo using micro-CT and 3D-image conversion with the MIMICS 14.0 software. As shown in the 3D images (FIG. 12), bone formation in the radial topography group occurred along the periphery of the bone defect and regeneration occurred along the direction of pattern. Interestingly, after 4 wk. and 6 wk., compact bone formation was not observed in the defect group or flat group of mice, whereas bone regeneration was significantly enhanced in the radial patterned scaffold group after 4 wk. and 6 wk. of implantation compared to that in the commercial products group. New bone formation was observed from the edge to center, depending on the radial direction. At 6 wk., the bone volume was 2.11 mm³ in the defect group, 2.67 mm³ in the flat patterned scaffolds group, 3.52 mm³ in the aligned scaffold group, 3.85 mm³ in the commercial products group, and 4.45 mm³ in the radial patterned scaffolds group (FIG. 13). The bone area was 29.81 mm² in the defect group, 39.78 mm² in the flat patterned scaffold group, 52.12 mm³ in the aligned scaffold group, 59.98 mm³ in the commercial product group, and 71.55 mm³ in the radial patterned scaffold group (FIG. 13). To analyze bone regeneration efficacy from radial topography, we performed H&E staining 6 wk. after implantation (FIG. 14). The histological analysis also confirmed greater bone formation-with a dense cytoplasm-in the radial patterned scaffolds compared to that in the other pattern, even the commercial products scaffolds. These results regarding bone regeneration and formation provide insights into the importance of radial topographical cues for inducing bone tissue regeneration.

### (4) Radial topographical cues on cellular migration and growth factor secretion

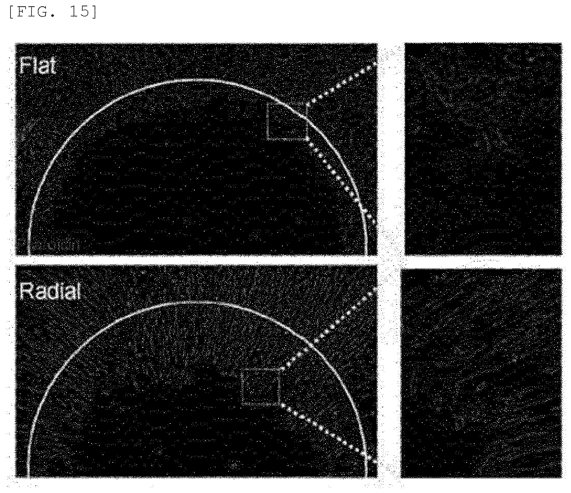

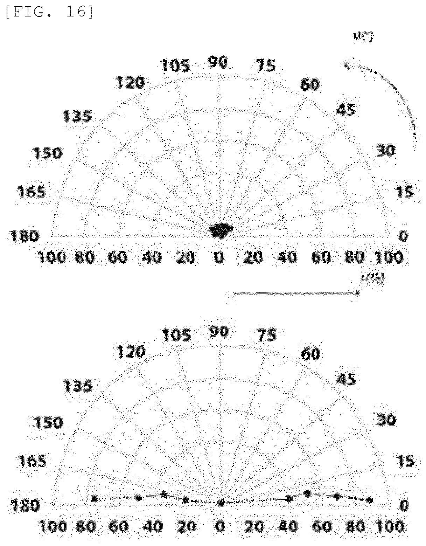

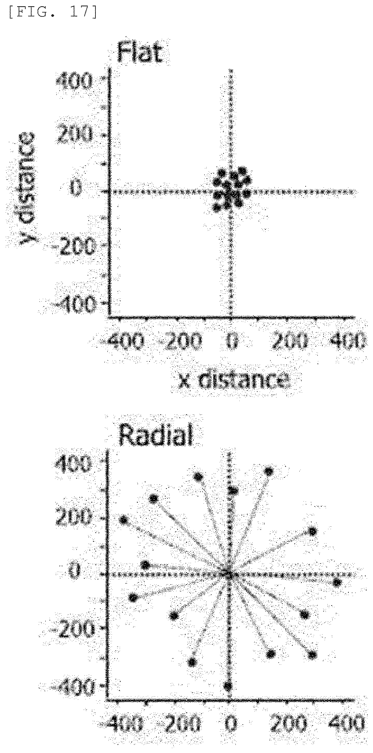

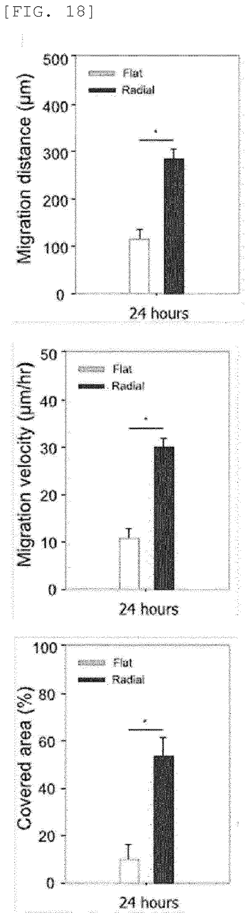

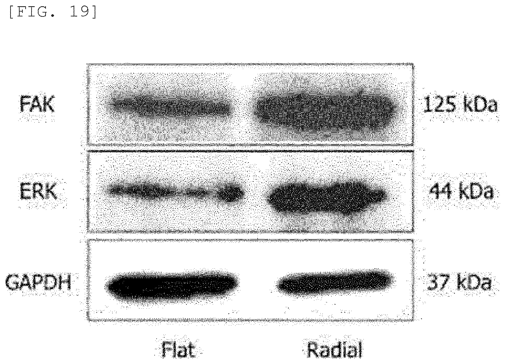

To explore a possible mechanism on the enhanced bone regeneration in the radial topographical cues, we investigated interaction between the cells and radial topographical scaffolds in terms of cellular migration and growth factor secretion. To generate a cell-free area on the PCL scaffolds for the cell migration assays, PDMS sheets (5 mm in diameter) were placed as a mask at the center of the PCL scaffolds (FIG. 15). We then mimicked the in vivo process of bone repair in the calvaria defects using the in vitro migration assays. After MG-63 cells were seeded and the PDMS sheets removed, cells were allowed to migrate toward the empty area at the center of the PCL scaffolds for 24h. The orientation of the MG-63 cells at 24h was then evaluated by phalloidin staining (FIG. 15). Interestingly, the cells on the radial patterned PCL scaffolds exhibited aligned morphologies arranged along the orientation of the radial topography. In contrast, there was no alignment of the cells on the flat PCL scaffolds. Quantification of the cell alignment on the radial topography showed that 85% of the cells were affected by topographical cues and aligned along the orientation of the radial topography (FIG. 16). Quantification of cell migration was also analyzed for the flat and radial topographies (FIG. 17). The results showed enhanced migratory behaviors of cells grown on the radial topography compared to those grown on the flat topography. The migration distance (280 um) and migration velocity (29 µm/h) of cells grown on the radial topography were higher than those of cells grown on the flat topography (115 µm and 12 µm/h, respectively) (FIG. 18). Similarly, the cell-free area was determined after cell migration for 24h. Migrating cells covered 55% of the area the on the radial topography, whereas only 12% of the area was covered by cells on the flat topography. To verify whether the radial topography could affect cellular function (e.g., proliferation and differentiation), migration-related signaling, and focal adhesion-related signaling, the expression of ERK and FAK was investigated. The ERK and FAK protein levels were evaluated by western blotting. The results revealed upregulation of ERK and FAK in cells cultured on the radial patterned scaffolds compared to that in cells cultured on the flat patterned scaffolds (FIG. 19).

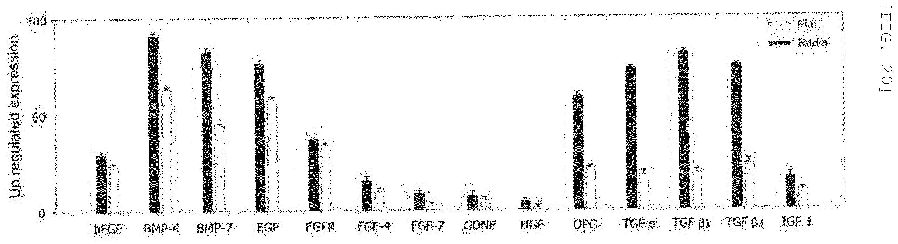

Next, the secretion of growth factor from osteoblast like cells was analyzed from cells cultured on the radially topography. The osteoblast like cells on the radially topography secreted higher levels of growth factors such as basic fibroblast growth factor (bFGF), bone morphogenetic protein-4 (BMP-4), bone morphogenetic protein-7 (BMP-7), epidermal growth factor (EGF), epidermal growth factor receptor (EGFR), fibroblast growth factor-4 (FGF-4), fibroblast growth factor-7 (FGF-7), glia cell-derived neurotrophic factor (GDNF), hepatocyte growth factor (HGF), osteoprotegerin (OPG), transforming growth factor alpha, beta1, and beta3 (TGF α, TGF β1 and TGF β3) and insulinlike growth factor 1(IGF-1) compared to flat topography (FIG. 20).

## Claims

1. A scaffold for tissue regeneration comprising:
a radial pattern which comprises a plurality of grooves and ridges on one surface thereof.

2. The scaffold for tissue regeneration according to claim 1, wherein the radial pattern is not formed in a central region of the scaffold.

3. The scaffold for tissue regeneration according to claim 1, wherein the radial pattern has a width of 0.1 um to 100 µm.

4. The scaffold for tissue regeneration according to claim 1, wherein a space between the radial patterns is 0.1 um to 100 µm.

5. The scaffold for tissue regeneration according to claim 1, wherein the scaffold has a circular shape.

6. The scaffold for tissue regeneration according to claim 1, the scaffold comprises: a substrate; and a pattern layer having the pattern on the substrate.

7. The scaffold for tissue regeneration according to claim 1, wherein the pattern layer includes at least one material selected from the group consisting of a polycaprolactone (PCL) resin, a polyurethane acrylate (PUA) resin, a polyvinyl alcohol resin, a polyethylene resin, a polypropylene resin, a polyethylene glycol resin, a poly(L-lactide-co-glycolide) resin, a polylactic acid (PLA) resin, a polyglycolic acid (PGA) resin, chitosan, gelatin, collagen, and a combination thereof.

8. A method of manufacturing the scaffold for tissue regeneration according to claim 1, the method comprising:
coating a glass with a polymer at a predetermined thickness;
pressing the coated polymer with a mold on which a radial pattern is formed to form a pattern; and
separating the polymer from the glass after the pattern is formed.

9. The method according to claim 8, wherein the coating is spin coating in which the polymer is placed on the glass, and then the polymer is adhered to the glass by rotating the glass.

10. The method according to claim 8, wherein the step of forming the pattern comprises:
heating the coated polymer; and
pressing the heated polymer with the mold having a radial pattern formed thereon, followed by cooling the same.

11. The method according to claim 8, further comprising:
heating the coated polymer before the step of forming the pattern;
pressing the heated polymer with a flat mold to flatten a surface thereof; and
cooling the flattened polymer.

12. The method according to claim 10 or 11, wherein the cooling is performed by spraying air to the glass.

13. The method according to claim 8, wherein the separating step is performed by immersing the polymer coated glass in ethanol.

14. The method according to claim 8, further comprising drying the separated polymer.
